## Europäisches Patentamt
## European Patent Office
## Office européen des brevets

(11) Publication number: **0 092 414**
**B1**

# EUROPEAN PATENT SPECIFICATION

(45) Date of publication of patent specification: **04.12.85**

(21) Application number: **83302178.5**

(22) Date of filing: **18.04.83**

(51) Int. Cl.⁴: **A 61 L 17/00**

(54) **Medical material.**

(30) Priority: **19.04.82 JP 65054/82**

(43) Date of publication of application:
**26.10.83 Bulletin 83/43**

(45) Publication of the grant of the patent:
**04.12.85 Bulletin 85/49**

(84) Designated Contracting States:
**DE FR GB IT NL SE**

(56) References cited:
**EP-A-0 051 354**
**FR-A-2 318 189**

**CHEMICAL ABSTRACTS, vol. 71, no. 17, 20th October 1969, page 165, no. 78956b, Columbus, Ohio, USA A.A. BAEV et al.: "Importance of macromolecular interaction in the biology of connective tissue"**

**The file contains technical information submitted after the application was filed and not included in this specification**

(73) Proprietor: **KOKEN CO. LTD.**
**5-18 Shimoochiai 3-chome**
**Shinjuku-ku Tokyo (JP)**

(72) Inventor: **Miyata, Teruo**
**6-29, Shimoochiai 3-chome**
**Sinjuku-ku Tokyo (JP)**
Inventor: **Noishiki, Tasuharu**
**Yamada, Misasa-cho**
**Touhaku-gun Tottori-ken (JP)**

(74) Representative: **Crampton, Keith John Allen et al**
**D YOUNG & CO 10 Staple Inn**
**London WC1V 7RD (GB)**

Courier Press, Leamington Spa, England.

## Description

This invention relates to medical materials that are compatible with living bodies, and with their production.

Medical materials used in direct contact with blood, such as artificial blood vessels and artificial heart valves, encounter the extremely serious problem of thrombus development.

Materials containing collagen, such as animal carotid arteries and human umbilical veins have been conventionally used for this purpose. It is known that artificial vessels making use of such materials are susceptible to development of thrombi and are thus often obstructed in the initial stages of their use, i.e., when blood is brought into contact with the surfaces of such artificial vessels, which surfaces are formed of foreign bodies other than endothelial cells, the blood immediately forms thrombi. It is thus most important that blood-contacting organs such as artificial blood vessels, artificial valves and artificial hearts should suppress thrombus formation and if possible achieve the formation of pseudo-endotheliums.

Much intensive research has been carried out to suppress thrombus formation, but no ideal antithrombotic medical materials have yet been developed.

As a result of experiments on collagen-containing materials such as those described above, it has been unexpectedly discovered that heparinized collagen, which has been formed by fixing protamine, a strongly basic protein, on collagen and then binding heparin to the protamine, can be used as a medical material having excellent antithrombotic properties and is therefore extremely effective for use in artificial blood vessels. It is also useful as a membrane having anti-adhesion effect. Heparin is an acidic polysaccharide having an inhibitory effect on blood coagulation.

The present invention provides a medical material comprising heparinized collagen in which heparin is bound to protamine fixed on collagen.

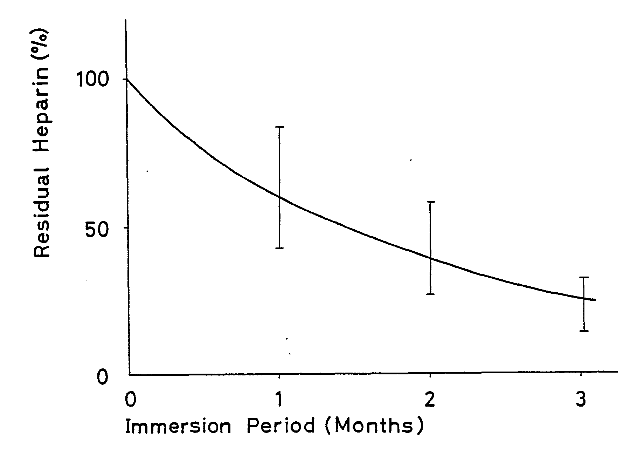

The single figure of the accompanying drawing shows the relationship between immersion period and residual heparin (96) when an artificial vessel made according to this invention was immersed in a physiological saline.

One example of the medical material according to this invention is formed by covering a base, which is made of such a synthetic polymer as described below, by collagen which has been heparinized via protamine as will be described below. As the synthetic polymer, may be mentioned polyesters such as Tetoron (registered Trade Mark). When a base made of such a synthetic polymer is kept inserted subcutaneously in an animal, the connective tissue (consisting principally of collagen) of the animal adheres around the base. Thus, by forming the above-described base with a tube of Tetoron mesh, a Tetoron mesh tube bearing a tubular connective tissue is obtained. It may be used as a starting artificial material for medical materials according to this invention.

Among starting materials useful in the practice of this invention may be mentioned tissues and organs making up living bodies, for example, animal carotid arteries, ureters, umbilical veins, paricardia and connective tissue pipes and natural materials derived from human tissues, as well as artificial materials containing collagen such as collagen tubes, collagen membrane, collagen sponges, collagen nonwoven fabric and collagen yarns.

The heparinization of collagen may be carried out in the following manner. First of all, one of the above-described collagen-containing natural and artificial materials is immersed in an aqueous solution of protamine so as to impregnate collagen fibers with protamine. After dipping the thus protamine-impregnated material in an aqueous solution of glutaraldehyde, it is immersed further in an aqueous solution of heparin to provide a medical material containing heparinized collagen.

The above-mentioned aqueous solution of protamine may be an aqueous solution of protamine sulfate or another protamine salt. The concentration of protamine in such an aqueous solution may range from 1 wt.% to its saturation. Immersion in this aqueous solution is carried out at a temperature in the range of 5—50°C and for 1—10 minutes.

The dipping in the aqueous solution of glutaraldehyde is effected at a temperature in the range of 5—50°C and for 1—20 minutes, using an aqueous solution which contains 0.1—5 wt.% of glutaraldehyde.

On the other hand, the concentration of the aqueous heparin solution may range from 0.01 wt.% to 10 wt.% and, preferably, from 0.5 wt.% to 3 wt.%. The immersion in the aqueous heparin solution is carried out at a temperature in the range of 5—50°C, at pH 2—10 and preferably at pH 4—8, and for 1—3 days.

Following the above procedures, it is ensured that sufficient heparin is bound to protamine. In other words, bonds are formed between positively-charged protamine and negatively-charged heparin. Free heparin, which is not bound to protamine, is removed by washing the heparin-treated material with water for 3 days.

It has been found through an *in vitro* test that medical materials containing the thus-heparinized collagen exhibit slow elution speeds of heparin in physiological saline and they permit elution of heparin over long periods of time. Therefore, such medical materials may be used as artificial organs which are brought into contact with blood, for example, artificial vessels, artificial valves and patching materials for cardiovascular organs. As a result of an experiment in which dogs were used, it has been found that they do not develop thrombi over long periods of time from the initial periods of their implantations and exhibit stable antithrombotic property. It has also been uncovered that medical materials containing collagen heparinized in such a manner as mentioned above are effective as anti-adhesion membranes.

The present invention will next be described in detail by the following examples.

### Example 1

A tube of 7 mm in inner diameter and 5.7 cm in length was formed with a Tetoron mesh. The tube was kept inserted subcutaneously for 20 days in an adult dog and then taken out as a connective tissue tube together with connective tissues attached around the mesh. The connective tissue tube was then treated overnight with a phosphate buffer (pH 7.0) which contained 0.01% of ficin. After washing the thus-treated tube with a physiological saline, it was immersed for 1 minute in an aqueous solution of protamine sulfate (concentration of protamine: 3%). It was further dipped for 5 minutes in a 1% aqueous solution of glutaraldehyde to fix protamine. After heparinizing the tube by immersion at 50°C and for 2 days in an aqueous solution of heparin (concentration: 1%, pH 6), the thus-heparinized tube was washed for 3 days with water while changing the water 3 to 5 times a day. The thus-obtained heparinized connective tissue tube was employed as an artificial vessel for a part of the thoracic descending aorta of a dog. No thrombus was adhered at all on the artificial vessel from the very beginning and the artificial vessel did not develop any obstruction as a stable and antithrombotic artificial vessel over a long period of time.

### Example 2

Surrounding fatty tissues were removed from a pig ureter. The resultant ureter was immersed overnight in a phosphate buffer (pH 7.0) containing 0.01% of ficin. Subsequent to washing the ureter with water, it was dipped for 3 minutes in an aqueous solution of protamine sulfate (concentration of protamine: 5%). It was further dipped for 5 minutes in a 1% aqueous solution of glutaraldehyde to fix protamine. After heparinizing the ureter by immersion at 50°C and for 2 days in an aqueous solution of heparine (concentration: 1%, pH: adjusted to pH 6 with acetic acid), the thus-heparinized ureter was washed for 3 days with water while changing the water 3—5 times a day.

The thus-obtained, heparinized pig ureter was cut into a 5 cm-long sample. The sample was immersed in 1 liter of a physiological saline maintained at room temperature. The physiological saline was changed once every week and the amount of heparin in the sample was investigated along the passage of time. The quantitative analysis of heparin was carried out by measuring the sulfur in heparin by means of an X-ray microanalyzer. In the diagram of the drawing, the ratios of measured heparin quantities to the initial heparin quantity are plotted as residual heparin (%) against their corresponding immersion periods. As readily envisaged from the diagram, the heparinized pig ureter of this Example retained as much as about 25% of the initial heparin even upon an elapsed immersion time of 3 months. Thus, it is understood that the elusion speed of heparin is slow and the heparinized pig ureter has a long-lasting antithrombotic effect.

The above-described heparinized pig ureter was used as an artificial vessel for a part of the thoracic descending aorta of a dog. This artificial vessel did not develop any thrombus from the beginning and exhibited stable antithrombotic property over a long period of time.

## Claims

1. A medical material comprising heparinized collagen in which heparin is bound to protamine fixed on collagen.

2. A medical material according to Claim 1, in which said medical material comprises a base made of a synthetic polymer and a connective tissue covering the base, and the heparinized collagen is present as collagen of the connective tissue.

3. A medical material according to Claim 2, in which the base is formed of a tube of a polyester mesh.

4. A medical material according to Claim 2 or 3, in which the connective tissue has been formed by subcutaneously implanting the base made of the synthetic polymer in an animal.

5. A medical material according to Claim 1, in which the medical material is formed of a natural tissue or organ derived from a living body and the heparinized collagen is present as collagen of the natural tissue or organ.

6. A medical material according to Claim 5, in which the natural tissue is a ureter.

7. A medical material according to Claim 1 formed of an artificial material that contains collagen, in which the heparinized collagen is present as the collagen of the artificial material.

8. An artificial blood vessel or an organ that is brought into contact with blood, made of a material according to any one of Claims 1 to 7.

9. A method of producing a medical material containing heparinized collagen, comprising immersing (a) a natural tissue or organ derived from a living body, or (b) an artificial material, in an aqueous solution of protamine, dipping the resultant material in an aqueous solution of glutaraldehyde, and then immersing the thus-treated material in an aqueous solution of heparin.

10. A method according to Claim 9, in which the immersion in the aqueous solution of protamine is carried out at a temperature in the range of 5—50°C and for 1—10 minutes, using an aqueous solution containing protamine at a concentration of from 1 wt.% to its saturation.

11. A method according to Claim 9 or 10, in which the material is dipped in an aqueous solution containing 0.1—5 wt% of glutaraldehyde at a temperature in the range of 5—50°C and for 1—20 minutes.

12. A method according to Claim 9, 10 or 11, in which the material is immersed in an aqueous solution containing 0.01—10 wt% of heparin at a temperature in the range of 5—50°C, at pH 2—10 and for 1—3 days.

13. A method according to Claim 12, in which the aqueous solution contains 0.5—3 wt% of heparin and the immersion is carried out at pH 4—8.

14. A method according to any one of Claims 9 to 13, in which the protamine fixed on the collagen has been obtained by reacting protamine impregnated in an stuck to the collagen with glutaraldehyde.

15. A process according to any one of Claims 9 to 14, in which item (a) is a natural tissue derived from a ureter.

16. A process according to any one of Claims 9 to 14, in which item (b) is a tube obtained by subcutaneously implanting a tube made of a polyester mesh in an animal and then taking out the tube together with connective tissue as a connective tissue tube.

## Revendications

1. Un matériel médical comprenant du collagène hépariné dans lequel de l'héparine est liée à de la protamine fixée sur du collagène.

2. Un matériel médical selon la revendication 1, où ledit matériel médical comprend une base, faite d'un polymère synthétique, et un tissu conjonctif recouvrant la base, et le collagène hépariné est présent sous forme du collagène du tissu conjonctif.

3. Un matériel médical selon la revendication 2, dans lequel la base est formée d'un tube d'un filet de polyester.

4. Un matériel médical selon la revendication 2 ou 3, dans lequel le tissu conjonctif a été formé par implantation sous-cutanée de la base faite du polymère synthétique à un animal.

5. Un matériel médical selon la revendication 1, où le matériel médical est formé d'un tissu ou organe naturels dérivés d'un organisme vivant et le collagène hépariné est présent sous forme du collagène du tissu ou de l'organe naturels.

6. Un matériel médical selon la revendication 5, dans lequel le tissu naturel est un uretère.

7. Un matériel médical selon la revendication 1, formé d'un matériel artificiel qui contient du collagène dans lequel le collagène hépariné est présent sous forme du collagène du matériel artificiel.

8. Un vaisseau sanguin ou un organe artificiel qui est mis en contact avec le sang, fait d'un matériel selon l'une quelconque des revendications 1 à 7.

9. Un procédé de production d'un matériel médical contenant du collagène hépariné, comprenant l'immersion (a) d'un tissu ou organe naturels dérivés d'un organisme vivant ou (b) d'un matériel artificiel, dans une solution aqueuse de protamine, le trempage de la matière obtenue dans une solution aqueuse de glutaraldéhyde puis l'immersion du matériel ainsi traité dans une solution aqueuse d'héparine.

10. Un procédé selon la revendication 9, dans lequel l'immersion dans la solution aqueuse de protamine est effectuée à une température dans la gamme de 5—50°C et pendant 1—10 min en utilisant une solution aqueuse contenant de la protamine à une concentration de 1% en poids de sa saturation.

11. Un procédé selon la revendication 9 ou 10, dans lequel on trempe le matériel dans une solution aqueuse contenant 0,1—5% en poids de glutaraldéhyde à une température dans la gamme de 5—50°C et pendant 1—20 min.

12. Un procédé selon la revendication 9, 10 ou 11, dans lequel le matériel est immergé dans une solution aqueuse contenant 0,01—10% en poids d'héparine à une température dans la gamme de 5—50°C à pH 2—10 et pendant 1—3 jours.

13. Un procédé selon la revendication 12, dans lequel la solution aqueuse contient 0,5—3% en poids d'héparine et l'immersion est effectuée à pH 4—8.

14. Un procédé selon l'une quelconque de revendications 9 à 13, dans lequel la protamine, fixée sur le collagène, a été obtenue par réaction de protamine imprégnant le collagène et y adhérant avec du glutaraldéhyde.

15. Un procédé selon l'une quelconque des revendications 9 à 14, dans lequel l'article (a) est un tissu naturel dérivé d'un uretère.

16. Un prodédé selon l'une quelconque des revendications 9 à 14, dans lequel l'article (b) est un tube obtenu par implantation sous-cutanée d'un tube fait d'un filet de polyester à un animal, puis le prélèvement du tube avec du tissu conjonctif comme tube de tissu conjonctif.

## Patentansprüche

1. Medizinisches Material enthaltend heparinisiertes Kollagen, bei dem das Heparin an Protamin gebunden ist, welches an Kollagen fixiert ist.

2. Medizinisches Material nach Anspruch 1, dadurch gekennzeichnet, daß das medizinische Material ein Substrat aus einem synthetischen Polymer und ein das Substrat bedeckendes Bindegewebe umfaßt, wobei das heparinisierte Kollagen als Kollagen des Bindegewebes vorliegt.

3. Medizinisches Material nach Anspruch 2, dadurch gekennzeichnet, daß das Substrat in Form eines Rohrs aus einem Polyestergeflecht vorliegt.

4. Medizinisches Material nach Anspruch 2 oder 3, dadurch gekennzeichnet, daß das Bindegewebe durch subkutanes Implantieren des Substrats aus dem synthetischen Polymer in ein Tier gebildet worden ist.

5. Medizinisches Material nach Anspruch 1, dadurch gekennzeichnet, daß es aus einem von einem lebenden Körper stammenden natürlichen Gewebe oder Organ abgeleitet ist und das heparinisierte Kollagen als Kollagen des natürlichen Bindegewebes oder Organs vorliegt.

6. Medizinisches Material nach Anspruch 5, dadurch gekennzeichnet, daß das natürliche Gewebe ein Harnleiter ist.

7. Medizinisches Material nach Anspruch 1, dadurch gekennzeichnet, daß es aus einem künstlichen Kollagen enthaltenden Material besteht, wobei das heparinisierte Kollagen als Kollagen des künstlichen Materials vorhanden ist.

8. Künstliches Blutgefäß oder Organ, welches mit Blut in Kontakt kommt, bestehend aus einem Material nach einem der Ansprüche 1 bis 7.

9. Verfahren zur Herstellung eines heparinisiertes Kollagen enthaltenden medizinischen Materials, dadurch gekennzeichnet, daß man (a) ein von einem lebenden Körper abgeleitetes natürliches Gewebe oder Organ oder (b) ein künstliches Material in eine wäßrige Lösung von Protamin eintaucht, das erhaltene Material in eine wäßrige Glutaraldehyd-Lösung eintaucht und dann das in dieser Weise behandelte Material in eine wäßrige Heparinlösung eintaucht.

10. Verfahren nach Anspruch 9, dadurch gekennzeichnet, daß das Eintauchen in die wäßrige Protaminlösung bei einer Temperatur im Bereich von 5 bis 50°C während 1 bis 10 Minuten durchgeführt wird, wobei eine wäßrige Lösung eingesetzt wird, die Protamin in einer Konzentration von 1 Gew.-% bis zur Sättigung enthält.

11. Verfahren nach Anspruch 9 oder 10, dadurch gekennzeichnet, daß das Material während eine Zeitdauer von 1 bis 20 minuten bei einer Temperatur im Bereich von 5 bis 50°C in eine 0,1 bis 5 Gew.-% Glutaraldehyd enthaltende wäßrige Lösung eingetaucht wird.

12. Verfahren nach Anspruch 9, 10 oder 11, dadurch gekennzeichnet, daß das Material während 1 bis 3 Tagen bei einem pH-Wert von 2 bis 10 und einer Temperatur im Bereich von 5 bis 50°C in eine 0,01 bis 10 Gew.-% Heparin enthaltende wäßrige Lösung eingetaucht wird.

13. Verfahren nach Anspruch 12, dadurch gekennzeichnet, daß die wäßrige Lösung 0,5 bis 3 Gew.-% Heparin enthält und das Eintauchen bei einem pH-Wert von 4 bis 8 durchgeführt wird.

14. Verfahren nach einem der Ansprüche 9 bis 13, dadurch gekennzeichnet, daß das an Kollagen fixierte Protamin durch Umsetzen des das Kollagen imprägnierenden und daran anhaftenden Protamins mit Glutaraldehyd gebildet wird.

15. Verfahren nach einem der Ansprüche 9 bis 14, dadurch gekennzeichnet, daß als Bestandteil (a) ein von einem Harnleiter abgeleitetes natürliches Gewebe eingesetzt wird.

16. Verfahren nach einem der Ansprüche 9 bis 14, dadurch gekennzeichnet, daß als Bestandteil (b) ein Rohr eingesetzt wird, welches durch subkutanes Implantieren eines Rohrs aus einem Polyestergeflecht in ein Tier und Entnehmen des Rohrs zusammen mit dem Bindegewebe als Bindegeweberohr erhalten worden ist.